# EUROPEAN PATENT APPLICATION

(11) **EP 3 838 276 A1**
(43) Date of publication of application: **23.06.2021**
(21) Application number: 19306662.8
(22) Date of filing: 17.12.2019
(51) Int. Cl.: A61K 31/60, A61K 35/74, A61P 1/04, A61P 29/00

(54) **ASSOCIATION OF FAECALIBACTERIUM PRAUSNITZII STRAIN CNCM I-4573 WITH PENTASA® FOR THE TREATMENT AND PREVENTION OF GASTROINTESTINAL INFLAMMATION**

(71) Applicant: Exeliom Biosciences, 21000 Dijon (FR); Institut national de recherche pour l'agriculture, l'alimentation et l'environnement, 75007 Paris (FR)
(72) Inventor: The designation of the inventor has not yet been filed
(74) Representative: Nony

(57) **Abstract**

The present invention relates to an association of a bacterial strain of the species *Faecalibacterium prausnitzii* deposited with the CNCM under accession number CNCM I-4573 with mesalamine, or a derivative thereof, and in particular to the use of this association in the treatment and/or prevention of an inflammatory gastrointestinal disease in an individual.

## Description

### Field of the invention

The present invention relates to the association of a bacterial strain of the species *Faecalibacterium prausnitzii* deposited with the CNCM under accession number CNCM 1-4573 with mesalamine or a derivative thereof, in particular for its use in the treatment and prevention of inflammatory gastrointestinal diseases (IBD) in an individual, in particular inflammatory bowel diseases (IBD).

### Prior art

Inflammation is a natural biological process which constitutes a normal part of the response to lesions or infections and contributes to protecting the organism against internal or external attacks.

However, a dysfunction of the inflammation mechanisms, in particular a persistent or excessively abundant inflammation, may cause painful diseases and endanger the patient's life. Such diseases comprise, for example, skin disorders, bowel disorders, neurological disorders, arthritis and autoimmune diseases. Several of these inflammatory diseases still have no treatment or have no appropriate treatment.

Consequently, the study of and search for new anti-inflammatory treatment strategies constitutes a major subject in medicine and in biomedical research.

An inflammatory bowel disease is a group of disorders characterized by a chronic and recurrent inflammation of the gastrointestinal tract. The most common form of this group is Crohn's disease. The pathogenesis involves inappropriate and continuous activation of the mucosal immune system caused by the presence of the intestinal microbiota in a genetically predisposed patient.

Aminosalicylic acid (in particular 5-ASA, also called mesalamine or mesalazine) or its derivatives thereof have been used successfully for a long time in the treatment of, in particular, ulcerative colitis and Crohn's disease.

A problem in the treatment with mesalamine is that it raises renal toxicity concerns and thus cannot be administered in too high quantities. Indeed, the incidence of renal impairment in patients with IBD treated with 5-ASA is estimated to be one in 100 patients, and interstitial nephritis occurs in one in 500 patients

Moreover, 5-ASA is known for being efficient in mild to moderate diseases forms, but also for being significantly less effective in more severe forms of the diseases. Accordingly, when 5-ASA is identified as being inefficient, the only available alternative consists in the use of immunosuppressive therapies. However, such therapies are quite expensive and are associated with potentially severe side effects such as infection and neoplasia.

Accordingly, there remains a need for an alternative solution to immunosuppressive therapies when 5-ASA is not sufficient enough in the treatment and/or prevention of inflammatory gastrointestinal diseases. There in particular remains a need for a novel solution allowing to improve the properties of mesalamine and its derivatives thereof in the treatment and/or prevention of inflammatory gastrointestinal diseases. There is more generally a constant need for novel substances, or combination of substances, for the treatment and/or prevention of inflammatory gastrointestinal diseases, in particular inflammatory bowel diseases, in an individual.

The present invention provides a solution to these problems.

### Summary of the invention

The objective of the present invention is to describe a new association of substances, and compositions comprising it, for the treatment and/or prevention of inflammatory gastrointestinal diseases in an individual, in particular inflammatory bowel diseases in an individual.

In the context of the present invention, the term "prevent" denotes the reduction to a lesser degree of the risk or of the probability of occurrence of a given phenomenon, that is to say, in the present invention, a gastrointestinal inflammation, in particular an inflammatory bowel disease. The term "prevent" must also be understood as encompassing the reduction to a lesser degree of the risk or of the probability of relapse of a disease according to the present invention, in particular of an inflammatory bowel disease.

In the context of the present invention, the term "treat", in particular when used in relation with a disease, denotes slowing down or stopping the development or progression of the disease, causing regression of the associated clinical symptoms or relieving the disease partially or completely. The term "treat" must also be understood as encompassing the reduction of number of relapses and/or the reduction of intensity of the relapses and/or the increase of the time separating two relapses of a disease according to the present invention, in particular of an inflammatory bowel disease.

The present invention is based on the discovery, by the present inventors, that when mesalamine, or a derivative thereof, is used in association with the *Faecalibacterium prausnitzii* strain CNCM 1-4573, this leads to an improvement of its anti-inflammatory properties.

In particular, the present inventors discovered that when mesalamine, or a derivative thereof, is used in a low ineffective quantity when alone, its administration to an individual in association with the *Faecalibacterium prausnitzii* strain CNCM 1-4573, also in an inefficient quantity when used alone, is unexpectedly able to provide anti-inflammatory properties.

According to the experimental results of the inventors, the association of mesalamine, or a derivative thereof, with a specific strain of *Faecalibacterium prausnitzii* (*F. prausnitzii*)*,* deposited with the CNCM under accession number CNCM 1-4573 on January 31, 2012, has the unexpected capacity to synergistically reduce a gastrointestinal inflammation, in particular a bowel inflammation, in an individual.

According to a first subject, the present invention accordingly relates to an association of a bacterial strain of the species *Faecalibacterium prausnitzii* deposited with the CNCM under accession number CNCM 1-4573 with mesalamine or a derivative thereof.

Derivatives of mesalamine can for example be selected from the group consisting of Mesalazine, Olsalazine, 4-ASA (4-Aminosalicylic acid) and Sulfasalazine.

Another object of the present invention is a pharmaceutical composition comprising, in a pharmaceutically acceptable medium, an association of the invention.

The invention further relates to an association according to the invention for use in the treatment and/or prevention of an inflammatory gastrointestinal disease in an individual.

An individual according to the invention is preferably a mammal, including a non-human mammal, and is, in particular, a human being.

The inflammatory gastrointestinal disease is, in particular, an inflammatory bowel disease (IBD), more particularly a colonic inflammatory bowel disease.

Said colonic inflammatory bowel disease may, in particular, be chosen from the group consisting of Crohn's disease, ulcerative colitis and pouchitis, in particular Crohn's disease and ulcerative colitis.

According to another embodiment, the association for use according to the invention is in a pharmaceutically acceptable medium of a pharmaceutical composition, preferably in an oral composition, and more particularly preferably in a pharmaceutical product.

The term "*pharmaceutically acceptable medium*" is intended to denote a medium which is compatible with the body of the individual to whom said composition must be administered. It is, for example, a non-toxic solvent such as water. In particular, said medium is compatible with oral administration.

A composition of the invention is preferably for oral administration.

A composition of the invention for oral administration may be chosen from the group consisting of a food product, a beverage, a pharmaceutical product, a nutraceutical, a food additive, a food supplement and a milk product.

### Figures' legends

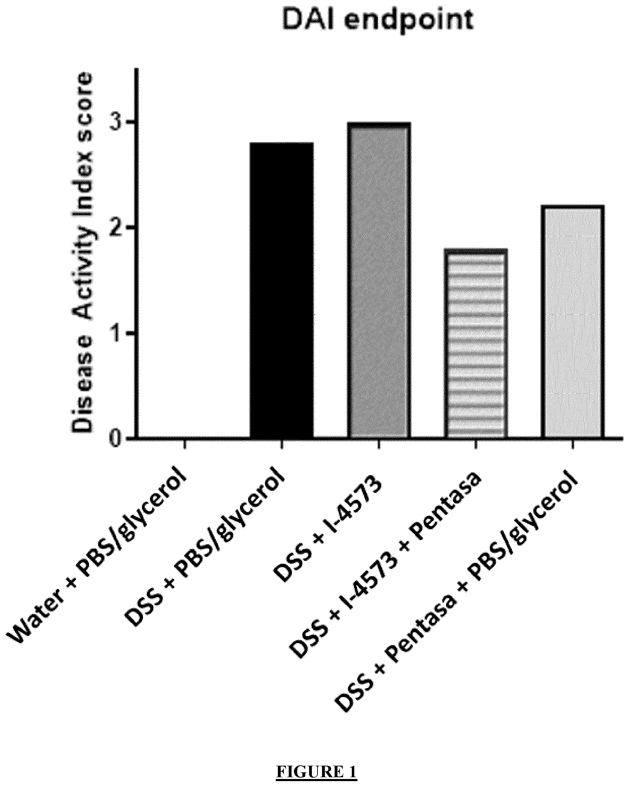

Figure 1 illustrates the Disease Activity Index (DAI) score obtained with, from the left to the right on the abscissa axis, (i) control mice (without colitis) receiving only water and PBS with glycerol; (ii) control mice having a DSS-induced colitis and treated with PBS with glycerol; (iii) mice having a DSS-induced colitis and treated with the *F*. *prausnitzii* strain CNCM 1-4573; (iv) mice having a DSS-induced colitis and treated with the association of *F*. *prausnitzii* strain CNCM 1-4573 and Pentasa®; and (v) mice having a DSS-induced colitis and treated with Pentasa® and PBS with glycerol. The DAI score is evaluated on a scale of 0 (no inflammation) to 4 (ordinate) (n=10/group except for Water+PBS/glycerol n=5).

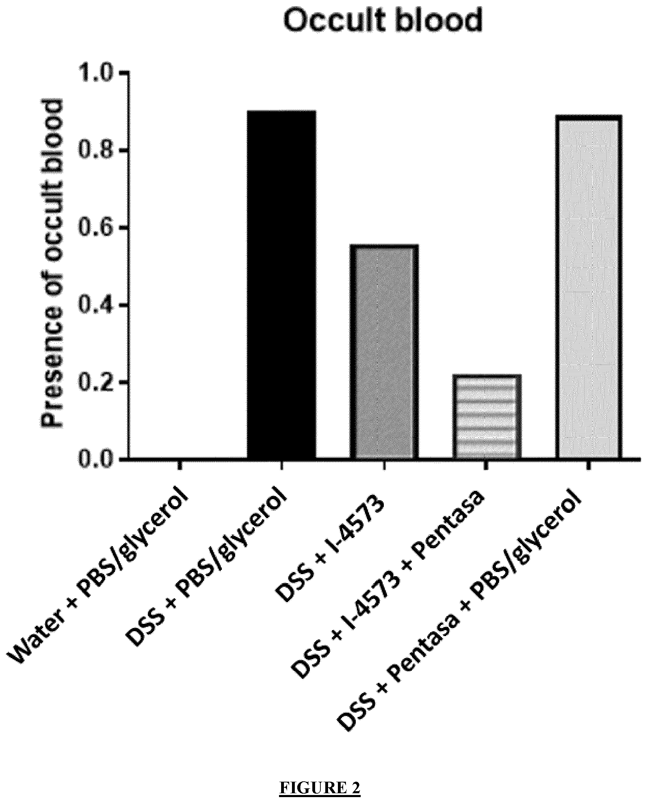

Figure 2 illustrates the presence of blood in the faeces of, from the left to the right on the abscissa axis, (i) control mice (without colitis) receiving only water and PBS with glycerol; (ii) control mice having a DSS-induced colitis and treated with PBS with glycerol; (iii) mice having a DSS-induced colitis and treated with the *F*. *prausnitzii* strain CNCM 1-4573; (iv) mice having a DSS-induced colitis and treated with the association of *F*. *prausnitzii* strain CNCM 1-4573 and Pentasa®; and (v) mice having a DSS-induced colitis and treated with Pentasa® and PBS with glycerol. The higher the quantity of blood present in the faeces, the higher the inflammation and the damages in the considered individual. The blood occurrence is rated according to criteria 0 (absence) or 1 (presence) (n=10/group except for Water+PBS/glycerol n =5).

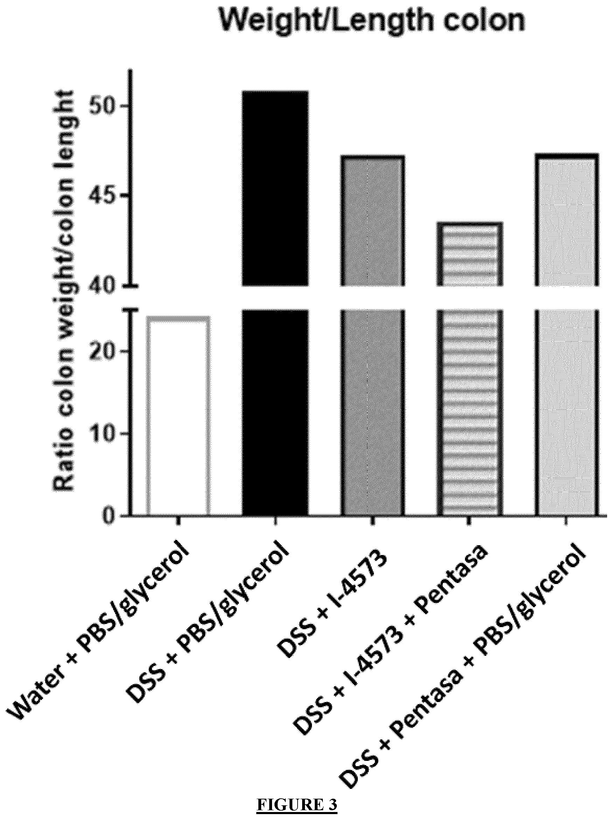

Figure 3 illustrates the ratio colon weight/colon length of, from the left to the right on the abscissa axis, (i) control mice (without colitis) receiving only water and PBS with glycerol; (ii) control mice having a DSS-induced colitis and treated with PBS with glycerol; (iii) mice having a DSS-induced colitis and treated with the *F*. *prausnitzii* strain CNCM 1-4573; (iv) mice having a DSS-induced colitis and treated with the association of *F*. *prausnitzii* strain CNCM 1-4573 and Pentasa®; and (v) mice having a DSS-induced colitis and treated with Pentasa® and PBS with glycerol. A decrease in size of the colon is induced by severe inflammation and an increase of colon weight is also observed due to edema/inflammatory infiltrate caused by severe inflammation.

### Detailed description of the invention

The present inventors have carried out thorough studies in order to identify the capacity of the association of a specific strain of *Faecalibacterium prausnitzii* with mesalamine or a derivative thereof, to synergistically treat and/or prevent inflammatory gastrointestinal diseases in an individual, in particular inflammatory bowel diseases in an individual.

Indeed, the inventors have determined, unexpectedly, that the *F*. *prausnitzii* strain deposited with the CNCM under accession number CNCM 1-4573 in association with mesalamine or a derivative thereof provides a better capacity to reduce a gastrointestinal inflammation, in particular a bowel inflammation, in an individual than the same agents used alone.

### Faecalibacterium prausnitzii strain

*F*. *prausnitzii* is a major member of the phylum *Firmicutes* and is part of the commensal bacteria that are the most abundant in the microbiota of the healthy human large intestine.

*F*. *prausnitzii* is an extremely oxygen-sensitive (EOS) bacterium which is therefore difficult to culture, even under anaerobic conditions (Duncan et al. 2002, Int. J. Syst. Evol. Microbiol. 52(Pt 6): 2141-6 and Lopez-Siles et al. Appl. Environ Microbiol. January 2012; 78 (2):420-8). *F*. *prausnitzii* is, in particular, known to be one of the most abundant butyrate-producing bacteria in the human digestive tract, the butyrate short-chain fatty acid being very important in intestinal physiology, systemic functions and beneficial effects on human health (Macfarlane and Macfarlane (2011), J. Clin. Gastroenterol. 45 Suppl: S120-7).

The *F*. *prausnitzii* strain A2-165 is also known to have anti-inflammatory and protective effects in murine models of acute and chronic colitis, that is to say in inflammatory disorders (Martin et al., Inflamm Bowel Dis. March 2014; 20(3):417-30 and Sokol et al., Proc Natl Acad Sci USA. October 28, 2008; 105(43):16731-6).

Moreover, WO2017129515 described the capacity of the *Faecalibacterium prausnitzii* strain deposited with the CNCM under accession number CNCM 1-4573 to reduce, *in vitro* and *in vivo*, a gastrointestinal inflammation, in particular a bowel inflammation, in an individual when used alone.

The anti-inflammatory properties of the A2-165 and 1-4573 strains cannot generally be attributed to the *F*. *prausnitzii* species, given that the existence of anti-inflammatory properties is unpredictable for a given strain of *F*. *prausnitzii.* Indeed, such a specific anti-inflammatory activity is illustrated in WO2017129515, in which a comparative test was carried out with the CNCM 1-4575 *F*. *prausnitzii* strain which does not have an anti-inflammatory activity.

A suitable daily dose of a bacterial strain according to the invention can be from 10³ to 10¹¹ bacteria in a composition of the invention, for example in the form of a daily dose equivalent to 10⁹ bacteria. The number of bacteria in a composition can be easily measured by the man skilled in the art using, for example, flow cytometry.

A bacterial strain according to the invention can be in a live, semi-active, inactivated or dead form, or a mixture thereof.

According to a particular embodiment, the bacterial strain according to the invention is used in a live or semi-active form, and in particular live form.

In another embodiment, the bacterial strain according to the invention is used in a inactivated or dead form.

An "inactivated" bacterial strain according to the invention is a bacterial strain that is no longer capable, temporarily, of forming colonies in culture. According to the present invention, a "dead" bacterial strain is a bacterial strain that is no longer capable, definitively, of forming colonies in culture. Dead or inactivated bacterial strains may have intact or ruptured cell membranes. Thus, the term "inactivated" also denotes bacterial strain extracts and lysates as detailed hereinbelow. Dead or inactivated bacterial strains may be produced via any method known to those skilled in the art.

An inactivated bacterial strain as mentioned above may be prepared by irradiation, heat inactivation, pH inactivation, inactivation by air or oxygen exposure, or lyophilization of a bacterial strain preparation. These methods are known to those skilled in the art.

More particularly, the inactivation of bacterial strains by irradiation may comprise the use of gamma rays, x-rays, exposure to UV or heat, or a pressure reduction.

Inactivation by lyophilization may be performed via any method known in the field. Advantageously, bacterial strains inactivated by lyophilization may be recultured.

Heat inactivation may be performed by incubating the bacterial strains of the invention for a prolonged period of time, for example at least two hours, at 170° C. Heat inactivation may also be performed by autoclaving, by subjecting the bacterial strains of the invention to a temperature of 121° C for a period of at least 20 minutes and at an atmospheric pressure of 2 bar. We include also heat inactivation process such as pasteurization, tyndallisation and so on.

Alternatively, the heat inactivation may be performed by subjecting the bacterial strains to a freezing temperature, for a prolonged period of time.

pH inactivation may be performed by incubation the bacterial strains of the invention for a prolonged period of time in a low or high pH, for example 30 min at pH2.

Inactivation by air exposure may be performed as *Faecalibacterium prausnitzii* is an Extremely Oxygen Sensitive (EOS) organism. The process may be done by incubated for a period of time the subjecting bacteria at air atmosphere.

A bacterial strain according to the invention may be used in whole form, i.e. essentially in its native form, or in the form of extracts or lysates of disintegrated suspensions comprising fractions and/or metabolites of this bacterium.

The term "metabolite" denotes any substance derived from the metabolism of the bacteria, and especially secreted by the bacteria under consideration according to the invention and also endowed with efficacy for the treatment and/or prevention of impairments of the skin's microrelief.

For the purposes of the invention, the term "fraction" more particularly denotes fragments of the said bacterium.

An extract or lysate that is suitable for use in the invention may be prepared from the bacterial strains at the end of the growth phase.

A bacterial strain that is suitable for use in the invention may be used in the form of a lysate.

For the purposes of the invention, a "lysate" commonly denotes a material obtained after the destruction or dissolution of biological cells via a phenomenon known as cell lysis, thus giving rise to the release of the intracellular biological constituents naturally contained in the cells of the bacteria under consideration.

For the purposes of the present invention, the term "lysate" is used without preference to denote the whole lysate obtained via lysis of the bacteria under consideration or only a fraction thereof. The lysate used is thus totally or partially formed from the intracellular biological constituents and from the constituents of the cell walls and membranes of the bacteria.

In a particular embodiment, a lysate used for the invention may be the whole lysate obtained via lysis of the bacterial strains under consideration.

This cell lysis may be accomplished via various known techniques, such as an osmotic shock, a heat shock, via ultrasonication, or alternatively under a mechanical stress of centrifugation type. In particular, a lysate of the invention may be obtained via ultrasonic disintegration of a medium comprising bacterial strains of the invention in suspension in order to release therefrom the cytoplasmic fractions, the cell wall fragments and the products derived from metabolism. All the components in their natural distribution are then stabilized in a weakly acidic aqueous solution.

The bacterial strain of the invention may be administered to the intestines of an individual to be treated in various ways, and in particular orally or rectally. A bacterium from an association of the invention is preferably administered orally.

According to one preferred embodiment, the bacterial strain of an association of the invention is included in a composition comprising a physiologically acceptable medium. Such a composition is preferably for oral administration.

### Mesalamine or a derivative thereof

Mesalamine represents the active compound aminosalicylic acid, and is also termed 5-ASA or mesalazine.

A mesalamine derivative in particular refers to its pharmaceutically acceptable salts, pharmaceutically acceptable solvates, pharmaceutically acceptable hydrates, pharmaceutically acceptable enantiomers, pharmaceutically acceptable derivatives and pharmaceutically acceptable polymorphs thereof.

Mesalamine and mesalamine derivatives have been marketed in different kinds of dosage forms, such as for example Pentasa® by Ferring and Lialda® by Shire, or Asacol®, Asacol HD® and Delzicol® by Warner-Chilcott. There also exist Apriso® by Salix, Canasa® by Aptalis Pharma, Rowasa® enema by Meda Pharms, Fivasa® by Tillots Pharma France, Dipentum® by Pharmacia or Quadrasa® by Norgine.

In a particular embodiment, Pentasa® is used in an association, or composition, according to the invention.

The amount of mesalamine, or its derivatives, used in an association or composition of the present invention can be in the range from about 100 to about 8000 mg, and in particular from about 100 to about 5000 mg. In a particular embodiment, a composition of the invention comprises 400 mg, 600 mg or 800 mg of mesalamine or a derivative thereof.

In a particular embodiment, the amount of mesalamine, or its derivatives thereof, that can be administered daily to a patient can go from 1000 mg to 5000 mg.

A *F*. *prausnitzii* strain according to the invention can be formulated and/or administered to an individual in the same composition as mesalamine or a derivative thereof, or in a separate composition.

When in two separate compositions, the *F*. *prausnitzii* strain and mesalamine, or a derivative thereof, can be administered simultaneously (or at substantially the same time) or following one another, through the same or through different route(s). For example, one can be administered rectally while the other is administered orally. By administered following one another, it is meant that the two actives composing an association of the invention are administered within minutes of each other or within hours of each other.

In an embodiment of the invention, the *F*. *prausnitzii* strain and mesalamine, or a derivative thereof, are administered simultaneously, in the same composition, orally or rectally, preferably orally.

In another embodiment, the *F*. *prausnitzii* strain and mesalamine, or a derivative thereof, are administered simultaneously, in separate compositions, independently orally or rectally, preferably both orally.

In a further embodiment, the *F*. *prausnitzii* strain and mesalamine, or a derivative thereof, are administered following one another, in separate compositions, independently orally or rectally, preferably both orally.

### Compositions

The present invention also relates to a composition, in particular a pharmaceutical composition, comprising, in a pharmaceutically acceptable medium, at least an association according to the invention.

A composition according to the invention is intended for the digestive tract, in particular the intestines.

Consequently, a composition according to the invention is chosen from an oral or rectal composition. A composition of the invention is preferably an oral or rectal composition, more preferably an oral composition.

According to one embodiment, a composition of the invention is an oral composition, that is to say that it is intended for oral administration to a subject.

Such a composition may be in the form of a suspension, a tablet, a pill, a capsule, a granule or a powder.

The composition according to the invention for oral administration may be chosen from the group consisting of a food product, a beverage, a pharmaceutical product, a nutraceutical, a food additive, a food supplement or a milk product and is, in particular, a food supplement or a pharmaceutical product.

A food supplement or pharmaceutical product for oral administration may for example be present in capsules, gel capsules, soft capsules, tablets, sugar-coated tablets, pills, pastes, lozenges, gums, oral solutions or emulsions, a syrup or a gel.

According to one particular embodiment, a composition according to the invention is a pharmaceutical product.

Advantageously, a composition according to the invention, intended for oral administration, may be provided with a gastric-juice-resistant coating, in order to ensure that the bacterial strain and mesalamine, or a derivative thereof, of the invention included in said composition can pass through the stomach undamaged. The release of the active agents may thus take place for the first time in the upper intestinal tract.

A composition according to the invention may also comprise a sweetener, a stabilizer, an antioxidant, an additive, a flavoring agent and/or a dye.

The formulation thereof is carried out by means of the usual methods for producing sugar-coated tablets, gel capsules, gels, controlled-release hydrogels, emulsions, tablets or capsules.

In another embodiment of the invention, a composition containing the bacterial strain of the invention is administered intrarectally.

Preferably, a rectal administration is carried out in the form of a suppository, an enema or a foam.

In particular, a composition of the invention is suitable for the administration of a daily dose of the bacterial strain of the species *Faecalibacterium prausnitzii* CNCM I-4573 representing from 10³ bacteria to 10¹² bacteria as a medicament, in particular a daily dose representing from 10⁴ bacteria to 10¹² bacteria as a medicament, more particularly a daily dose representing from 10⁶ to 10¹² bacteria as a medicament, preferably in the form of a daily dose equivalent to 10¹⁰ bacteria (i.e. total cell count by cytometry).

In particular, a composition of the invention is suitable for the administration of a daily dose of the bacterial strain of the species *Faecalibacterium prausnitzii* CNCM 1-4573 representing from 10³ bacteria to 10¹² bacteria, in particular a daily dose representing from 10⁴ bacteria to 10¹² bacteria, more particularly a daily dose representing from 10⁶ to 10¹² bacteria, preferably in the form of a daily dose equivalent to 10¹⁰ bacteria (i.e. total cell count by cytometry).

For example, a composition according to the invention may be administered to an individual, at a single daily dose of 1 g containing the CNCM 1-4573 bacterial strain of the invention in an amount equivalent to a dose of between 10³ and 10¹² bacteria, in particular a dose of between 10⁴ and 10¹² bacteria, more particularly a dose of between 10⁶ and 10¹² bacteria, preferably 10¹⁰ bacteria.

In another example, a composition according to the invention may be administered to an individual requiring it, at a single daily dose of 0.2 g containing the CNCM 1-4573 bacterial strain of the invention in an amount equivalent to an amount of between 10³ and 10¹² bacteria, in particular a dose of between 10⁴ and 10¹² bacteria, more particularly a dose of between 10⁶ and 10¹² bacteria, preferably 10¹⁰ bacteria.

In another example, a composition according to the invention may be administered to an individual requiring it, twice a day on the basis of two doses of 1 g, each dose containing, independently, the CNCM 1-4573 bacterial strain of the invention in an amount equivalent to an amount of between 5x10³ bacteria and 5x10¹¹ bacteria, in particular between 5×10⁶ bacteria and 5×10¹¹ bacteria, preferably 5×10⁹ bacteria, so that the total daily dose of the CNCM 1-4573 bacterial strain of the invention administered to the individual is as indicated above.

A composition according to the invention may also comprise at least one among: antioxidants, fish oils, DHA, EPA, vitamins, minerals, phytonutrients, a protein, a lipid, probiotics, and combinations thereof.

The invention is described below in greater detail by means of the following examples which are given solely by way of illustration.

All the references to percentages are percentages by weight unless otherwise indicated.

### EXAMPLES

The *F*. *prausnitzii* strain deposited with the CNCM under the accession number CNCM 1-4573 and PENTASA®, were tested alone and in combination for their capacity to modulate, *in vivo*, the immune response and have a direct impact on bowel inflammation.

A murine DSS-induced colitis model is used in an *in vivo* study of the anti-inflammatory effects of these various elements.

More particularly, C57BL/6 mice (8-9 weeks old at the beginning of the assay) are kept at room temperature, under 12h light/dark cycles and have free access to food and water, except the day before the induction of colitis, where they are fasted for 12h. Colonic inflammation is induced by treatments with Dextran Sodium Sulfate (DSS). In details, DSS is dissolved in drinking water (2.5% wt/vol) from day 7 to day 12 and the animals are free to drink this solution for 5-days. Water consumption is measured in the DSS-treated groups and compared to groups of naive mice drinking water: no difference is observed for the volume of liquid consumed, between water and DSS-drinking mice. Mice are treated daily orally with 200 µL by gavage of:
- group 1: 1.10⁹ colony forming units (CFU) of *F*. *prausnitzii* strain CNCM 1-4573; and
- group 2: 1.10⁹ colony forming units (CFU) of *F*. *prausnitzii* strain CNCM 1-4573 + Pentasa®, which is mixed in the food *ad libitum* (2,88 g of Pentasa® granules (1 granule weighs 0.5 mg and contains 0.26 mg of 5-ASA; The mouse 5-ASA dose is 150 mg/day/kg body weight).

A third group (group 3) consists of DSS mice to which Pentasa® mixed in the food has been provided *ad libitum* with the same dosage as indicated for the group 2.

Two control groups are also studied:
- one group of non-colitis mice to which only PBS glycerol has been administered orally by gavage; and
- one group of DSS mice to which only PBS glycerol has been administered by gavage.

The treatments or vehicle are administrated orally by gavage, seven days (day 0) prior first DSS administration in preventive mode. The food with Pentasa® is also given to the mice of groups 2 and 3 as only food source from day 0. The mice in group 3 are gaved by PBS/glycerol in order to limit the bias introduced by gavage stress. The treatment period for each group continues to the end of the assay i.e. day 19.

At day 7, DSS is added to drinking water for 5 days (from day 7 to day 12). The necropsy is performed seven days after end of DSS period at day 19.

Body weight and survival rate are recorded daily from two days prior day 2 and until euthanasia at day 19.

Different parameters are monitored at euthanasia of the experiment to evaluate the effect of the products on colonic inflammation. The Disease Activity Index (DAI) is a simple scoring system used to determine the severity of colitis in mice. It is calculated based upon evaluation of body weight changes, stool consistency, and the presence of blood in the feces. Briefly, DAI was assessed by an investigator blinded to the protocol according to a standard scoring system. Body weight (BW), stool consistency (with a score from 0 - 3, 0=normal, 1= soft, 2= Diarrhea, 3=watery diarrhea, and visible presence of blood (rectum of mice) were recorded daily.

At euthanasia, the presence of Occult Blood (OB) is recorded using the hemoccult method. Loss in BW is scored as: 0, no weight loss; 1, weight loss of <10% from baseline; 2, >10%. For stool consistency, a score of 0 was assigned for well-formed pellets, 1 for pasty and semi formed stools that do not adhere to the anus, and 2 for liquid stools that adhere to the anus. For OB, a score of 0 is assigned for no blood, 1 for positive OB or for gross bleeding. These scores are added together.

The mice are sacrificed by cervical dislocation and the abdominal cavity is opened. The colon and the small intestine are removed. The colon length is measured and weighted to calculate a ratio weight/length of colon.

The results obtained are represented in Figures 1 to 3.

The results are presented in the form of bar graphs.

All comparisons are analysed using the Permutation Test for two independent samples, the most powerful statistical test adapted small number of samples. Statistics are calculated using the StatXact software (Cytel Inc, Cambridge, MA, USA). The comparison is performed against DSS group control.

When considering Figure 1, it can be seen that no statistical difference is observed for the DAI between the results obtained in DSS mice not treated or treated either with the F. prausnitzii strain according to the invention alone or with Pentasa® alone. On the contrary, when an association of the invention is administered to DSS mice, the observed DAI result is statistically decreased compared to the one of non-treated DSS mice.

The same applies regarding the Weight/Length of the colon results represented in Figure 3.

Concerning the results represented in Figure 2 and relating to the presence of blood in the faeces, it can be seen that no statistical difference is observed between the results obtained in DSS mice not treated and the results obtained with DSS mice treated with Pentasa® alone. While the F. prausnitzii strain according to the invention used alone shows a slight effect in reducing the occult blood in DSS mice (reduction of the present of occult blood from about 0.9 in non-treated DSS mice to about 0.6), the result obtained with an association of the invention administered to DSS mice is significantly higher (reduction of the present of occult blood from about 0.9 in non-treated DSS mice to about 0.2).

A synergistic effect of the association of strain *F*. *prausnitzii* CNCM 1-4573 with Pentasa® on the treatment of an inflammatory gastrointestinal disease has accordingly, and unexpectedly, been demonstrated by the present inventors.

## Claims

1. Association of a bacterial strain of the species *Faecalibacterium prausnitzii* deposited with the CNCM under accession number CNCM 1-4573 with mesalamine or a derivative thereof.

2. Association according to claim 1, wherein the mesalamine derivative is selected from the group consisting of Mesalazine, Olsalazine, 4-ASA (4-Aminosalicylic acid) and Sulfasalazine.

3. Pharmaceutical composition comprising, in a pharmaceutically acceptable medium, an association as defined in claim 1 or 2.

4. Association as defined in claim 1 or 2, for use in the treatment and/or prevention of an inflammatory gastrointestinal disease in an individual.

5. The association for use according to claim 4, wherein the individual is a mammal.

6. The association for use according to claim 4 or 5, wherein the individual is a human being.

7. The association for use according to any one of claims 4 to 6, wherein the inflammatory gastrointestinal disease is an inflammatory bowel disease.

8. The association use according to any one of claims 4 to 7, wherein the inflammatory gastrointestinal disease is a colonic inflammatory bowel disease.

9. The association for use according to claim 8, wherein the colonic inflammatory bowel disease is chosen from the group consisting of Crohn's disease, ulcerative colitis and pouchitis.

10. The association for use according to claim 8 or 9, wherein the colonic inflammatory bowel disease is chosen from the group consisting of Crohn's disease and ulcerative colitis.

11. The association for use according to any one of claims 4 to 10, wherein the association is in a pharmaceutically acceptable medium of a pharmaceutical composition.

12. The association for use according to claim 11, wherein the composition is suitable for the administration of a daily dose of the bacterial strain of the species *Faecalibacterium prausnitzii* deposited with the CNCM under accession number CNCM 1-4573 representing from 10³ bacteria to 10¹² bacteria, in particular a daily dose representing from 10⁴ bacteria to 10¹² bacteria, more particularly a daily dose representing from 10⁶ to 10¹² bacteria, preferably in the form of a daily dose equivalent to 10¹⁰ bacteria.

13. The association for use according to claim 11 or 12, wherein the composition is for oral administration, and is in particular chosen from the group consisting of a food product, a beverage, a pharmaceutical product, a nutraceutical, a food additive, a food supplement and a milk product.

14. The association for use according to claim 13, **characterized in that** the composition is a pharmaceutical product.
